# EUROPÄISCHE PATENTANMELDUNG

(11) **EP 3 610 786 A1**
(43) Veröffentlichungstag der Anmeldung: **19.02.2020**
(21) Anmeldenummer: 19179703.4
(22) Anmeldetag: 12.06.2019
(51) Int. Cl.: A61B 5/00, A61B 5/021

(54) **VORRICHTUNG ZUR BLUTDRUCKMESSUNG**

(30) Priorität: 15.08.2018 DE 102018006601
(71) Anmelder: seca gmbh & co. kg, 22089 Hamburg (DE)
(72) Erfinder: Vogel, Frederik, 20149 Hamburg (DE)
(74) Vertreter: Klickow & Wetzel PartGmbB

(57) **Zusammenfassung**

Die Erfindung betrifft eine Vorrichtung zur nicht-invasiven Messung des Blutdruckes eines Lebewesens, aufweisend mindestens ein Blutdruckmessgerät mit einer Messeinrichtung sowie einer Benutzerschnittstelle, ausgebildet als mindestens ein Touch-Display, und einen Barcode-Scanner zur Identifikation eines Patienten und/oder Bedieners. Die erfindungsgemäße Vorrichtung weist eine drahtlose Schnittstelle auf, über die die Vorrichtung mit einem Netzwerk verbindbar ist. Die Messung des Blutdruckes ist mit der Vorrichtung sowohl als Aufwärtsmessung als auch als Abwärtsmessung durchführbar. Weiterhin ist die erfindungsgemäße Vorrichtung zur Messung des Blutdruckes in einer vorteilhaften Ausführungsform für einen mobilen Einsatz nutzbar.

## Beschreibung

Die Erfindung betrifft eine Vorrichtung zur automatischen, nicht-invasiven Messung des Blutdruckes eines Lebewesens, aufweisend mindestens ein elektronisches Blutdruckmessgerät.

Zur Messung von Körpereigenschaften und Körperfunktionen von menschlichen Personen oder Tieren werden sowohl mobile als auch stationäre Messgeräte eingesetzt. Bei den zu messenden Eigenschaften oder Funktionen handelt es sich häufig um Bio- bzw. Körperimpedanzen, Herzfunktionen oder Nervenfunktionen. Weitere Messgrößen in diesem Zusammenhang können beispielsweise Körpergewicht, Körperzusammensetzungen, geometrische Abmessungen, Fingerabdrücke, Körpertemperaturen an verschiedenen Stellen, Zusammensetzung des verwerteten Atemgases, Atemgasvolumina oder Blutdruckwerte sein.

Insbesondere der Blutdruck eines Lebewesens ist ein wichtiger Indikator für den Gesundheitszustand. Im Rahmen von Krankheitsdiagnosen ist die Ermittlung des Blutdruckes daher ein Standardmessverfahren, das neben diversen anderen Messungen und Tests von Körpereigenschaften und/oder Körperfunktionen durchgeführt wird und als einzelner Messwert, eine Messwertkette pro Zeiteinheit und/oder in Verbindung mit weiteren Messwerten, die verschiedenen anderen Körpereigenschaften und Körperfunktionen betreffend, zur Diagnose von Krankheiten oder Risikozuständen herangezogen wird.

Unter dem Blutdruck eines Lebewesens versteht man grundsätzlich den Druck des Blutes in einem Blutgefäß, wobei der Druck technisch gesehen eine Flächenpressung als Resultat einer auf ein Flächenelement rechtwinkelig wirkenden Kraftkomponente darstellt. Der Blutdruck ist abhängig von der Herzleistung und dem Gefäßwiderstand und wird in mmHg (mm Quecksilbersäule) gemessen. Aufgrund der Tatsache, dass das Herz als Pumporgan diskontinuierlich arbeitet, ist der Blutdruck nicht mit einem einzigen Druckwert beschreibbar. Vielmehr müssen zwei Werte ermittelt werden: Der systolische (obere) Blutdruck, der entsteht, wenn das Herz kontrahiert und das Blut in die Blutgefäße drückt, und der diastolische (untere) Blutdruck, der sich einstellt, wenn sich der Herzmuskel in der Dehnungsphase befindet und sich mit Blut füllt.

Der Blutdruck kann basierend auf verschiedenen Blutgefäßen ermittelt werden: Arterien, Venen oder an Blutgefäßen mit besonderen Aufgabenstellungen wie zum Beispiel Schlagadern. Von besonderem Interesse sind die Blutdruckwerte in den Arterien, d.h. arterielle Blutdruckwerte, weil deren Ermittlung in besonders einfacher und risikoloser Weise durch indirekte Messverfahren möglich ist. Bei diesen indirekten Messverfahren erfolgt die Ermittlung des Blutdruckes von außerhalb des Körpers des Lebewesens, die deshalb auch als unblutige oder nicht-invasive Messverfahren bezeichnet werden. Die indirekte Blutdruckmessung wird durch stationäre oder mobile Messgeräte realisiert, die elektronisch oder mechanisch-manuell arbeiten. Die Messwerteaufnahmevorrichtung für die Blutdruckmessung besteht häufig aus einer Manschette, die als Vorrichtung zur Blutdruckmessung ausgeführt ist und den Kontakt zwischen der zugeordneten Messwerteverarbeitungs- und Auswertevorrichtung und dem Lebewesen herstellt.

Bei der indirekten Blutdruckmessung kommen verschiedene Messprinzipien zur Anwendung. Die Prinzipien werden üblicherweise durch manuelle Messungen realisiert und erfassen den arteriellen Blutdruck in auskultatorischer, palpatorischer oder oszillometrischer Weise. Dem Grunde nach basieren die verschiedenen Prinzipien auf der Erkenntnis, dass die innerhalb einer Arterie auftretenden Geräusche gemessen werden die auftreten, wenn diese Arterie zunächst abgeschnürt wird und anschließend wieder freigegeben wird.

Allen Prinzipien ist gemeinsam, dass die als Manschette ausgebildete Vorrichtung zur Abschnürung des Blutgefäßes an einer Extremität des Lebewesens festgelegt wird. Diese Vorrichtung zur Blutdruckmessung ist entweder schlauchförmig oder als zu einem Schlauch formbares bahn- und damit flächenförmiges Band mit einem Verschlussmechanismus ausgebildet und verfügt wenigstens abschnittsweise über ein Druckkissen. Das Druckkissen ist durch einen mit einem Medium befüllbaren, elastischen Hohlraum realisiert, der durch die druckbeaufschlagte Befüllung den Querschnitt vergrößert und dadurch eine für die Blutdruckmessung erwünschte Abschnürung der Extremität in Bezug auf die Blutzirkulation bewirkt.

Wird als Extremität für die Messung des Blutdruckes der Oberarm einer Person genutzt, bewirkt das Expandieren der Manschette eine Komprimierung der Oberarmarterie (Arteria brachialis) und daraus folgend Pulsationen, deren Oszillationen Schwankungen des Druckes in der Manschette auslösen. Aus dem Verlauf dieser Druckzustände können sowohl der systolische als auch der diastolische Blutdruck mit einer geeigneten Messvorrichtung digital bestimmt werden. Aus diesen beiden Werten kann zudem der mittlere arterielle Druck ermittelt werden.

Als nicht-invasive Blutdruckmessverfahren sind nach den genannten Prinzipien das klassische Abwärtsverfahren und das neuere Aufwärtsverfahren bekannt. In einem Abwärtsverfahren zur Messung des Blutdruckes wird das Druckkissen der Manschette auf einen Druck oberhalb des erwarteten systolischen Blutdrucks aufgepumpt, während der Messung schrittweise gesenkt und dabei der systolische sowie der diastolische Blutdruck bestimmt. In einem Aufwärtsverfahren zur Messung des Blutdruckes wird der Manschettendruck schrittweise soweit erhöht, bis der systolische Blutdruck stabil bestimmbar ist. Das Aufwärtsverfahren ist für einen Patienten aufgrund des geringeren auftretenden Manschettendruckes schonender und meist schneller abgeschlossen.

Wird als Messvorrichtung ein digitales Gerät mit einem Display genutzt, so lassen sich die Messdaten bzw. -ergebnisse auf diesem darstellen und einfach ablesen. Weiterhin kann ein solches elektronisches Messgerät in bekannten Ausführungsformen eine Automatik zum Aufblasen und Ablassen des druckbeaufschlagenden Mediums der Manschette aufweisen, sodass ein manuelles Pumpen, beispielsweise mithilfe eines Balgs, nicht erforderlich ist.

Die Verwaltung von Patientenakten erfolgt in Kliniken und Arztpraxen nach dem heutigen Standard meist digital. In Krankenhäusern werden die Patienten zudem in der Regel mithilfe von Barcodes, die die Patienten auf einem Band am Arm oder Bein tragen, identifiziert. Die Messdaten von medizinischen Mess- und/oder Diagnosegeräten werden in das digitale System eingespeist und in der jeweiligen Patientenakte abgespeichert. Verfügt ein medizinisches Mess- oder Diagnosegerät über keine eigene Schnittstelle, um die Messdaten direkt in das Verwaltungssystem zu übertragen, müssen die Daten manuell in das System eingepflegt werden, was zeitaufwändig und fehleranfällig ist.

Eine kabelgebundene Schnittstelle, beispielsweise eine USB-Schnittstelle, erfordert stets ein passendes Kabel zum Anschluss an ein an das digitale Verwaltungssystem angeschlossenes Gerät und ist anfällig für Verschmutzung.

Weiterhin müssen nach dem Stand der Technik die Messdaten und die Identität des Patienten separat ermittelt und zusammengeführt in das System eingepflegt werden.

Eine Aufgabe der Erfindung ist es, eine Vorrichtung zur Messung des Blutdruckes derart zu konstruieren, dass die Messdaten aus einer Blutdruckmessung eines Lebewesens schnell und einfach an ein weiterverarbeitendes System, wie zum Beispiel in die digitale Patientenakte eines Patienten in einem Krankenhaus oder in einen EHR bzw. EMR, übermittelbar sind.

Diese Aufgabe wird erfindungsgemäß dadurch gelöst, dass die Vorrichtung zur nicht-invasiven Messung des Blutdruckes über eine kabellose Schnittstelle mit einem Netzwerk verbindbar ist, über das die relevanten Daten beispielsweise in ein Verwaltungssystem für Patientenakten bzw. -daten überführbar sind.

Eine weitere Aufgabe der Erfindung ist es, die Verknüpfung der Messdaten mit der Identität eines Patienten und/oder eines Bedieners zur vereinfachen.

Diese Aufgabe wird erfindungsgemäß dadurch gelöst, dass die Vorrichtung zur Messung des Blutdruckes einen Barcode-Scanner aufweist, mit dem ein Barcode zur Identifikation eines Patienten und/oder eines Bedieners auslesbar und mit den Messdaten verknüpfbar ist.

Die Messdaten und die Identität eines Patienten können mithilfe der erfindungsgemäßen Vorrichtung zur Messung des Blutdruckes somit automatisiert noch auf dem Messgerät verbunden werden und direkt über eine drahtlose Schnittstelle an das weiterverarbeitende System übermittelt werden. Eine erfindungsgemäße Realisierung einer Vorrichtung zur Messung des Blutdruckes umfasst sowohl mobile als auch stationäre Messvorrichtungen.

Eine weitere Aufgabe der Erfindung ist es, die haptischen Eigenschaften einer mobilen Vorrichtung zur nicht-invasiven Blutdruckmessung derart zu verbessern, dass diese einhändig bedienbar ist.

Diese Aufgabe wird erfindungsgemäß dadurch gelöst, dass die mobile Einheit der Messvorrichtung einen Griffsteg aufweist, der einen an eine durchschnittliche Hand angepassten Umfang aufweist, sodass der Griffsteg angenehm und sicher mit den Fingern und der Handfläche einer Hand eines Bedieners ergriffen und gehalten werden kann, und dass die bedienrelevanten Elemente räumlich derart angeordnet sind, dass diese mit dem Daumen der das Gerät haltenden Hand betätigbar sind.

Eine vorteilhafte Ausführungsform einer erfindungsgemäßen Vorrichtung zur mobilen, nicht-invasiven Blutdruckmessung eines Lebewesens weist eine Basisstation und ein mobiles Blutdruckmessgerät, sowie einen Luftschlauch und eine Blutdruckmanschette auf.

Die Basisstation dient der Aufbewahrung des mobilen Geräts und ist beispielsweise als ein Tischständer ausgebildet. Eine integrierte Ladefunktionalität für den Akku der mobilen Einheit ist denkbar. In einer besonders vorteilhaften Ausführungsform ist ein separates externes Netzteil der mobilen Einheit derart an die Basisstation ankoppelbar, dass der Ladevorgang der mobilen Einheit durch das bloße Einstellen der mobilen Einheit in die Basisstation durchführbar ist.

Das mobile Gerät ist in einer vorteilhaften Ausführungsform als ein Handgerät ausgebildet und in ein Gehäuse aus einem schlag- und stoßfesten Kunststoff integriert. In einer besonders vorteilhaften Ausführungsform ist der Kunststoff weiterhin hautverträglich und selbstverlöschend. Die Gehäuseform ist in einem oberen Bereich, der ein Display beherbergt, breiter ausgedehnt und weist in einem unteren Bereich einen Griffsteg auf, der für eine sichere und angenehme Handhabbarkeit Gummi-Elemente aufweisen kann.

Die mobile Einheit weist mindestens eine Messeinheit zur nicht-invasiven Blutdruckmessung, die in einer besonders vorteilhaften Ausführungsform sowohl als Aufwärtsals auch als Abwärtsmessung vorgenommen werden kann, sowie mindestens eine Benutzerschnittstelle, besonders vorteilhaft ausgebildet als ein Touch-Display, und eine Schnittstelle zur kabellosen Anbindung an ein Netzwerk, insbesondere mittels WLAN, auf. Zusätzlich weist eine vorteilhafte Ausführungsform einer mobilen Einheit einer erfindungsgemäßen Vorrichtung einen Barcode-Scanner auf, der die Identifikation und Zuordnung eines Patienten und/oder Bedieners durch das Scannen eines individuellen Barcodes ermöglicht. Neben dem Touch-Display sind weitere Bedienelemente ausgebildet als Knöpfe oder Taster bzw. Schalter denkbar. Eine Kombination der erfindungsgemäßen Blutdruckmessvorrichtung mit einem Rollstativ ist denkbar.

Die mindestens eine Messeinheit der erfindungsgemäßen Vorrichtung ist in einer vorteilhaften Ausführungsform als ein digitales Blutdruckmessmodul ausgebildet, mit dem der Blutdruck eines Patienten nach dem oszillometrischen Prinzip, bevorzugt an dem Oberarm des Patienten, bestimmbar ist. Das Blutdruckmessmodul weist eine Pumpvorrichtung auf, mit der das Druckkissen der Manschette mit Druck beaufschlagbar ist und der Druck ablassbar ist. Der Manschettendruck ist mithilfe des digitalen Blutdruckmessmoduls überwachbar und wird in einem Bereich von 0 mmHg bis 300 mmHg geregelt. Neben der Pumpvorrichtung weist das Blutdruckmessmodul mindestens einen Drucksensor auf. In einer besonders vorteilhaften Ausführungsform werden mindestens zwei Druckkanäle überwacht.

Das Touch-Display ist in einer vorteilhaften Ausführungsform einer erfindungsgemäßen mobilen Einheit als ein Mehrfarben-TFT-Display ausgebildet, das eine resistive Touch-Einheit aufweist, um eine Bedienbarkeit mit OP-Handschuhen zu ermöglichen.

Die Schnittstelle zur kabellosen Anbindung an ein Netzwerk ist in einer vorteilhaften Ausführungsform der erfindungsgemäßen Blutdruckmessvorrichtung als ein WLAN-Modul ausgebildet.

Weitere elektronische Baugruppen in einem mobilen Gerät einer erfindungsgemäßen Ausführungsform einer Blutdruckmessvorrichtung sind ein Akku, der vorteilhaft als ein Lithium-Ionen- oder ein Lithium-Polymer-Akku ausgebildet ist, eine Spannungsversorgung, die die weiteren Module des mobilen Gerätes mit den benötigten Spannungen und Strömen versorgt und ein akustischer Signalgeber für akustische Ausgaben.

Mithilfe der erfindungsgemäßen Vorrichtung ist eine "Leise Messung" durchführbar, durch die eine besonders geräuscharme Messung des Blutdruckes bei sensiblen Patienten realisierbar ist. Gleichzeitig werden bei einer "Leise-Messung" die geltenden Normen über maximale Zeiten und Drücke eingehalten.

Im Folgenden wird die erfindungsgemäße Vorrichtung in beispielhafter Ausführungsform erläutert. Es zeigt:
- Fig. 1: eine perspektivische Ansicht einer erfindungsgemäßen Vorrichtung zur Messung des Blutdrucks schräg von vorn,
- Fig. 2: eine perspektivische Ansicht eines erfindungsgemäßen mobilen Gerätes zur Blutdruckmessung von vorn,
- Fig. 3: eine perspektivische Ansicht des mobilen Gerätes aus Fig. 2 von hinten,
- Fig. 4: eine perspektivische Ansicht des mobilen Gerätes aus Fig. 2 von der linken Seite,
- Fig. 5: eine perspektivische Ansicht des mobilen Gerätes aus Fig. 2 von unten und
- Fig. 6: ein Blockschaltbild der erfindungsgemäßen Vorrichtung zur Messung des Blutdrucks.

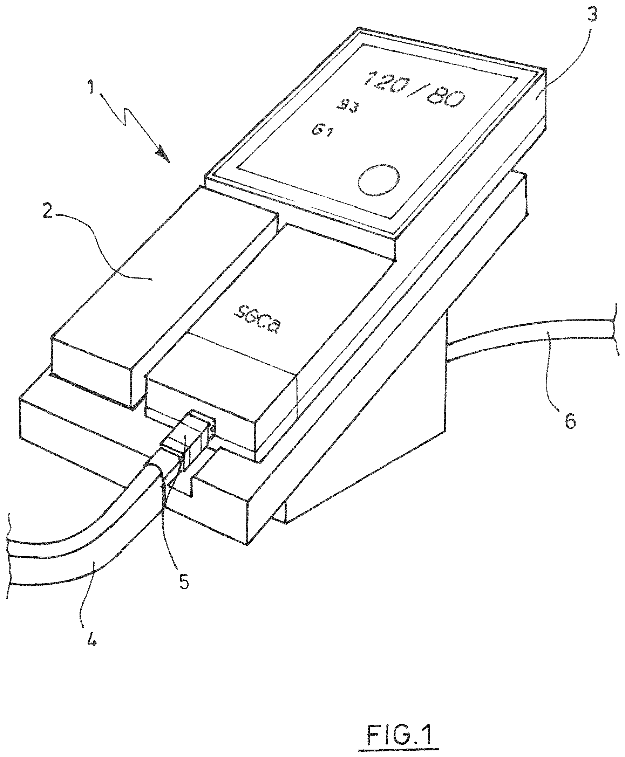

In Figur 1 ist eine vorteilhafte Ausführungsform einer erfindungsgemäßen Vorrichtung (1) zur Messung des Blutdruckes in einer perspektivischen Ansicht schräg von vorn dargestellt. Die Vorrichtung (1) weist eine Halterungsvorrichtung (2) in Form einer Basisstation ausgebildet als Tischständer sowie ein mobiles Blutdruckmessgerät (3) auf. An das mobile Blutdruckmessgerät (3) ist ein Schlauch (4) über eine Kupplung (5) angekoppelt, der dem Anschluss einer nicht dargestellten Blutdruckmessmanschette dient. Über ein Kabel (6) ist ein separates externes Netzteil an den Tischständer (2) angekoppelt, das das mobile Blutdruckmessgerät (3) mit Spannung versorgt.

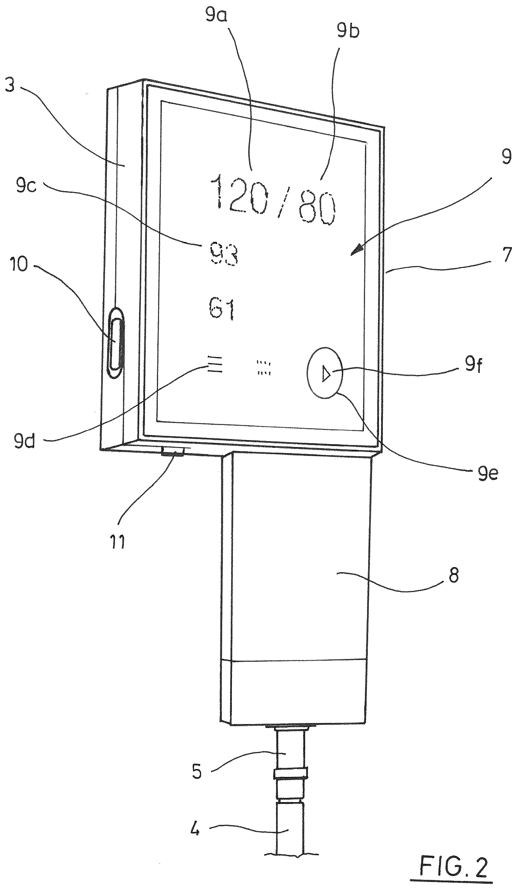

Figur 2 zeigt das mobile Blutdruckmessgerät (3) in einer perspektivischen Ansicht von vorn. In der gezeigten Ausführungsform ist das mobile Blutdruckmessgerät (3) in einen breiteren oberen Bereich (7) und einen schmaleren unteren Bereich (9) gegliedert. Der untere Bereich (7) ist als ein Griffsteg ausgebildet, der eine haptisch angenehme und sichere Handhabung des Blutdruckmessgerätes (3) ermöglicht und zugleich den Daumen eines Benutzers freigibt, um das Blutdruckmessgerät (3) zu bedienen. Im oberen Bereich (7) des mobilen Blutdruckmessgeräts (3) ist ein Touch-Display (9) angeordnet, das in dem dargestellten Betriebszustand die Messergebnisse in Form von systolischem (9a) und diastolischen Blutdruck (9b) eines Patienten in mmHg, den durchschnittlichen Blutdruck (9c) eines Patienten in mmHg, den Puls eines Patienten in Schlägen pro Minute und die Auswahlmöglichkeiten "Menü" (9d), "Start Barcode-Scan" (9e) und "Start einer Messung" (9f) anzeigt. Die Auswahlmöglichkeiten (9d, 9e, 9f) lassen sich aufgrund der erfindungsgemäßen Ausbildung des unteren Bereiches des Blutdruckmessgerätes (3) als Griffsteg (8) mit dem Daumen der das Blutdruckmessgerät (3) haltenden Hand eines Benutzers bedienen. In einem eingeschalteten Zustand ist somit eine einhändige Bedienung des erfindungsgemäßen mobilen Gerätes (3) möglich. An der linken Seite des Blutdruckmessgerätes (3) ist im oberen Bereich (7) ein Ein-/Aus-Schalter (10) angeordnet. An der unteren Seite des oberen Bereiches (7) ist eine Anschlussbuchse (11) für den Stecker eines nicht dargestellten externen Netzteils, das zum Laden des mobilen Blutdruckmessgerätes (3) dient. Auf der unteren Seite des Griffstegs (8) ist der zur Manschette führende Luftschlauch (4) mittig angekoppelt.

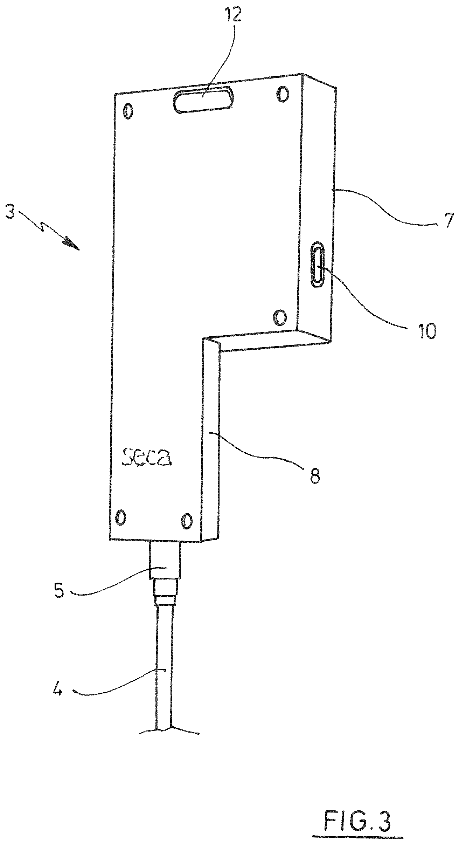

Figur 3 zeigt das erfindungsgemäße mobile Blutdruckmessgerät (3) in einer perspektivischen Ansicht von hinten. Rückseitig ist im oberen Bereich (7) des Blutdruckmessgeräts (3) mittig am oberen Ende ein Barcode-Scanner (12) angeordnet.

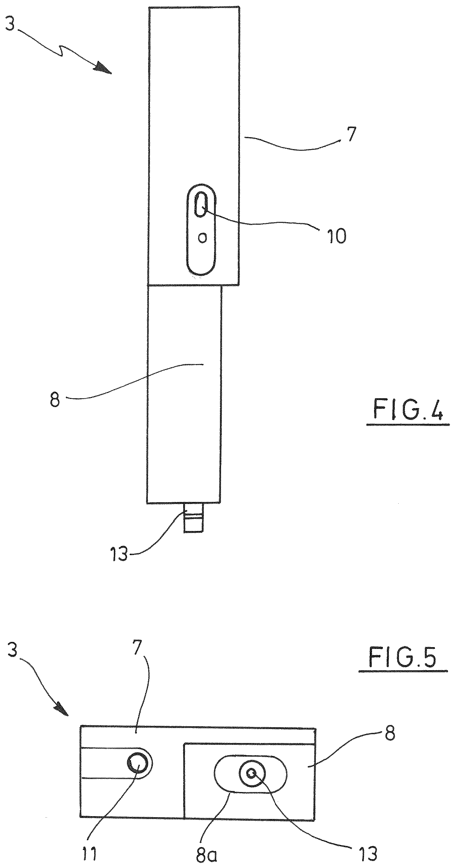

In Figur 4 ist das erfindungsgemäße mobile Blutdruckmessgerät (3) in einer Ansicht von der linken Seite dargestellt. An dem Kopplungsstück (13) am Griffsteg (8) kann ein Schlauch (4) mithilfe der Kupplung (5) angekoppelt werden.

Figur 5 zeigt das erfindungsgemäße mobile Blutdruckmessgerät (3) in einer Ansicht von unten. An der unteren Seite des oberen Bereichs (7) des mobilen Blutdruckmessgeräts (3) ist auf der linken Seite mittig die Anschlussbuchse (11) für das Netzkabel angeordnet. Mittig auf der unteren Seite des Griffstegs (8) befindet sich das Kopplungsstück (13) für den Schlauch (4). Die Kontur (8a) des Griffstegs (8) ist an den Seiten abgerundet und auf eine gute Haptik ausgelegt. Die Kontur des oberen Bereiches (7) des Blutdruckmessgerätes (3) ist auf der Rückseite abgerundet und auf der vorderen Seite flach gestaltet.

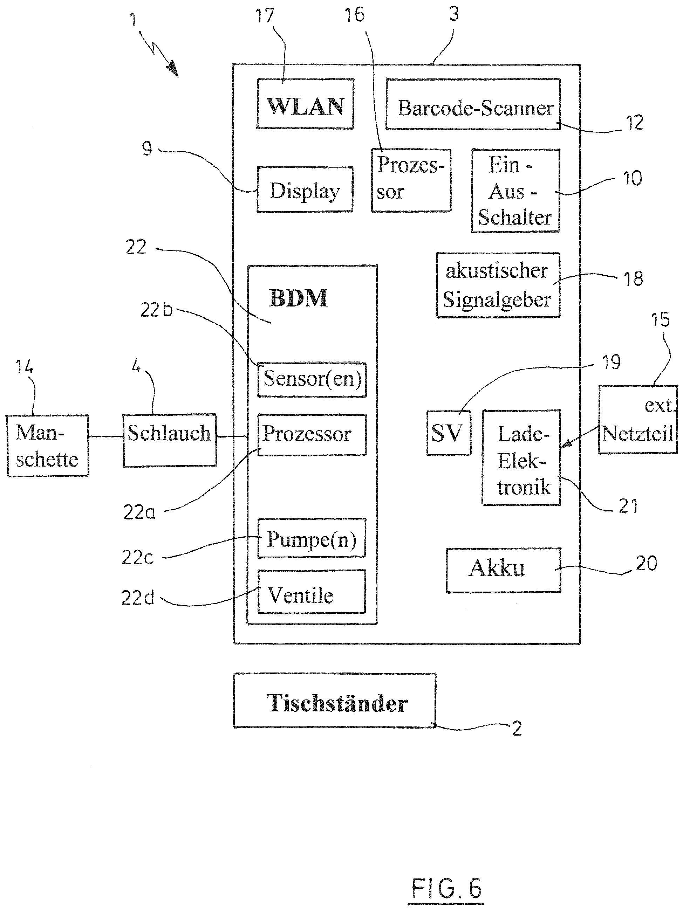

In Figur 6 ist ein Blockschaltbild der erfindungsgemäßen Vorrichtung zur Messung des Blutdruckes einer Person (1) dargestellt. An das mobile Blutdruckmessgerät (3) angeschlossen sind ein Schlauch (4), der das Blutdruckmessgerät (3) mit einer Blutdruckmessmanschette (14) verbindet, und ein externes Netzteil (15), das das mobile Blutdruckmessgerät (3) auflädt, wenn die Ladung des Energiespeichers des mobilen Blutdruckmessgeräts (3) unterhalb eines Schwellwertes gesunken ist.

Das mobile Blutdruckmessgerät (3) weist einen zentralen Prozessor (16) auf, mit dem die Steuerung des mobilen Blutdruckmessgeräts (3) realisierbar ist. Die kabellose Anbindung an ein Netzwerk ist über ein WLAN-Modul (17) herstellbar. Über das Touch-Display (9) sind Messergebnisse und Menüführung darstellbar und Benutzereingaben detektierbar. Der Ein-/Aus-Schalter dient zum Ein- und Ausschalten des mobilen Blutdruckmessgeräts (3). Mithilfe des Barcode-Scanners (12) sind Barcodes einlesbar und darüber Patienten und/oder Bediener identifizierbar. Für akustische Ausgaben weist das mobile Blutdruckmessgerät (3) in der dargestellten Ausführungsform einen akustischen Signalgeber (18) auf, der beispielsweise als Lautsprecher ausgebildet sein kann.

Für die Energieversorgung der einzelnen Baugruppen weist das mobile Blutdruckmessgerät (3) eine Spannungsversorgung (19) auf, die vorteilhaft als ein PMIC ausgebildet ist, um die verschiedenartigen Anforderungen der Lasten abdecken zu können. Im mobilen Betrieb ist das Blutdruckmessgerät (3) über einen integrierten Akku (20) mit Energie versorgbar, der vorteilhaft als Lithium-Ionen Akkumulator ausgebildet ist und austauschbar im Gehäuse des Blutdruckmessgeräts (3) verbaut ist.

In einer besonders vorteilhaften Ausführungsform weist das Blutdruckmessgerät (3) zum Laden des Akkus (20) eine Lade-Elektronik (21) auf, die die speziellen Anforderungen des Lithium-Ionen-Akkus (20) erfüllt. Weiterhin weist das mobile Blutdruckmessgerät (3) ein digitales Blutdruckmessmodul (22) auf, durch das mindestens ein Druckkanal, in besonders vorteilhafter Ausführungsform mindestens zwei Drückkanäle, mithilfe von Drucksensoren (22b) überwachbar sind.

Das Blutdruckmessmodul (22) weist einen Prozessor (22a) auf, auf dem die Steuerung der Messtechnik implementierbar ist. Zudem weist das digitale Blutdruckmessmodul (22) zum Aufpumpen bzw. Abpumpen des druckbeaufschlagenden Mediums der Blutdruckmessmanschette mindestens eine Pumpe (22c) und mindestens ein Ventil (22d) auf.

## Patentansprüche

1. Vorrichtung zur elektromechanischen, nicht-invasiven Messung des Blutdruckes (1), **dadurch gekennzeichnet, dass** diese über eine drahtlose Schnittstelle (17) mit einem Netzwerk verbindbar ist und dass diese einen Barcode-Scanner (12) aufweist.

2. Vorrichtung nach Anspruch 1, **dadurch gekennzeichnet, dass** mit dieser eine nicht-invasive Blutdruckmessung sowohl als Aufwärtsmessung als auch als Abwärtsmessung durchführbar ist.

3. Vorrichtung nach mindestens einem der vorstehenden Ansprüche, **dadurch gekennzeichnet, dass** diese eine Benutzerschnittstelle aufweist, die mindestens als ein Touch-Display ausgebildet ist.

4. Vorrichtung nach mindestens einem der vorstehenden Ansprüche, **dadurch gekennzeichnet, dass** die drahtlose Schnittstelle (17) zur Anbindung an ein Netzwerk als ein WLAN-Modul ausgebildet ist.

5. Vorrichtung nach mindestens einem der vorstehenden Ansprüche, **dadurch gekennzeichnet, dass** diese zumindest ein mobiles Blutdruckmessgerät (3) und eine Basisstation (2) aufweist.

6. Vorrichtung nach mindestens einem der vorstehenden Ansprüche, **dadurch gekennzeichnet, dass** diese ein mobiles Blutdruckmessgerät (3) aufweist, das einhändig zeitgleich haltbar und bedienbar ist.

7. Vorrichtung nach Anspruch 6, **dadurch gekennzeichnet, dass** die einhändige Bedienbarkeit durch die Ausbildung eines Bereiches des mobilen Blutdruckmessgerätes (3) als Griffsteg (8) und die Anordnung der bedienungsrelevanten Eingabevorrichtungen (9d, 9e, 9f) in einem mit dem Daumen eines Benutzers bei einem sachgerechten Halten des Blutdruckmessgerätes (3) erreichbaren Bereich realisiert ist.

8. Vorrichtung nach Anspruch 7, **dadurch gekennzeichnet, dass** die Eingabevorrichtungen (9d, 9e, 9f) als digitale Tasten auf dem Touch-Display (9) realisiert sind.

9. Vorrichtung nach mindestens einem der vorstehenden Ansprüche, **dadurch gekennzeichnet, dass** diese mit OP-Handschuhen bedienbar ist.

10. Vorrichtung nach mindestens einem der vorstehenden Ansprüche, **dadurch gekennzeichnet, dass** mit dieser ein Blutdruckmessverfahren als "Leise Messung realisierbar ist.

11. Vorrichtung nach mindestens einem der vorstehenden Ansprüche, **dadurch gekennzeichnet, dass** die Messwertermittlung des Blutdruckes auf dem oszillometrischen Prinzip beruht.

12. Vorrichtung nach mindestens einem der vorstehenden Ansprüche, **dadurch gekennzeichnet, dass** diese mobil einsetzbar ist.

13. Vorrichtung nach Anspruch 12, **dadurch gekennzeichnet, dass** die mobile Einsetzbarkeit der Vorrichtung (1) dadurch realisiert ist, dass das mobile Blutdruckmessgerät (3) als ein Handgerät ausgebildet ist.
